# EUROPEAN PATENT APPLICATION

(11) **EP 4 159 161 A1**
(43) Date of publication of application: **05.04.2023**
(21) Application number: 21818194.9
(22) Date of filing: 22.04.2021
(51) Int. Cl.: A61C 9/00, A61C 13/00, A61B 34/10, A61B 5/00, G16H 30/40, G16H 50/20

(54) **METHOD AND APPARATUS FOR PREDICTING MISSING TOOTH IN ORAL IMAGE**

(30) Priority: 01.06.2020 KR 20200065943
(71) Applicant: DIO Corporation, Busan 48058 (KR)
(72) Inventor: KIM, Jin Cheol, Busan 48058 (KR); KIM, Jin Baek, Busan 48058 (KR)
(74) Representative: Treeby, Philip David William
(86) International application number: PCT/KR2021/005097
(87) International publication number: WO 2021/246643

(57) **Abstract**

The present invention provides a method for predicting a missing tooth in an oral image, comprising the steps of: generating a standard tooth model by statistically learning a plurality of training images; whole-area-matching an oral image to the standard tooth model; and predicting a missing tooth by comparing first and second neighboring teeth located on both sides of the missing tooth, respectively, with first and second standard teeth of the standard tooth model corresponding to the first and second neighboring teeth, respectively.

## Description

### CROSS-REFERENCE TO RELATED APPLICATION

This application claims priority to and the benefit of Korean Patent Application No. 10-2020-0065943, filed on June 1, 2020, the disclosure of which is incorporated herein by reference in its entirety.

### [Technical Field]

The present invention relates to a method and apparatus for predicting a missing tooth in an oral image.

### [Background Art]

In general, when the original human tissue is lost, an implant means a substitute that can replace the human tissue, but in dentistry, it refers to implanting an artificially made tooth. In other words, it is a procedure that restores the function of teeth by implanting a fixture made of titanium or the like, which does not have a rejection reaction in the human body, in the alveolar bone from which the tooth has escaped, and then fixing an artificial tooth to replace the lost root.

The implant procedure is performed by forming a perforation in the alveolar bone and placing a fixture, and then coupling an abutment and a crown to the fixture. As such, there are many differences between implant procedures for each patient, and this is because the implant placement position, direction and depth are determined by considering various factors such as the patient's alveolar bone condition, the central position of a missing tooth requiring implant, the central axis direction and size and the like

However, the process of determining the central position, central axis direction and size of a missing tooth is quite difficult for beginners who do not have abundant surgical experience, as well as experienced dentists.

Recently, a computer program is used to design a crown that matches the missing tooth based on the patient's oral image, but even in this case, the situation is that determining the central position, central axial direction and size of a missing tooth in the oral image is dependent on the operator's experience.

### [Disclosure]

### [Technical Problem]

In order to solve the problems of prior art as described above, an object of the present invention is to provide a method and apparatus for predicting a missing tooth in an oral image, in which the central position, central axial direction and size information of a missing tooth are automatically detected to automatically place a crown to match a missing tooth area.

The technical problems to be achieved in the present invention are not limited to the technical problem mentioned above, and other technical problems that are not mentioned will be clearly understood by those of ordinary skill in the art to which the present invention pertains from the following description.

### [Technical Solution]

In order to solve the aforementioned problems, the present invention provides a method for predicting a missing tooth in an oral image, including the steps of generating a standard tooth model by statistically learning a plurality of training images, whole-area matching an oral image to the standard tooth model, and predicting a missing tooth by comparing first and second neighboring teeth located on both sides of the missing tooth, respectively, with first and second standard teeth of the standard tooth model corresponding to the first and second neighboring teeth, respectively.

In addition, the method for predicting a missing tooth in an oral image according to the present invention further includes the step of locally matching the oral image and the standard tooth model based on the first and second standard teeth.

Herein, the step of generating a standard tooth model is a step of generating the standard tooth model by learning a matching image obtained by matching an individual tooth model to an oral scan image.

In addition, the step of whole-area matching is a step of matching all individual teeth included in the standard tooth model as a group.

In addition, the step of predicting a missing tooth includes the step of predicting a central position of the missing tooth based on a first position deviation that is a central position difference between the first neighboring tooth and the first standard tooth and a second position difference that is a central position difference between the second neighboring tooth and the second standard tooth.

In addition, the step of predicting a central position of the missing tooth is a step of predicting a central position of the missing tooth by applying the first and second position deviations to the central position of a third standard tooth of the standard tooth model corresponding to the missing tooth and located between the first and second standard teeth.

In addition, the step of predicting a central position of the missing tooth is a step of predicting a central position of the missing tooth by applying an intermediate value of the first and second positional deviations to the central position of the third standard tooth.

In addition, the step of predicting a missing tooth includes the step of predicting a central axis of the missing tooth based on a first angular deviation that is an angular difference between the central axes between the first neighboring tooth and the first standard tooth and a second angular deviation that is an angular difference between the central axes between the second neighboring tooth and the second standard tooth.

In addition, the step of predicting a central axis of the missing tooth is a step of predicting a central axis of the missing tooth by applying the first and second angular deviations to the central axis of a third standard tooth of the standard tooth model corresponding to the missing tooth and located between the first and second standard teeth.

In addition, the step of predicting a central axis of the missing tooth is a step of predicting a central axis of the missing tooth by applying an intermediate value of the first and second angular deviations to the central axis of the third standard tooth.

In addition, the step of predicting a missing tooth includes a step of predicting a size of the missing tooth based on a first size deviation that is a difference in size between the first neighboring tooth and the first standard tooth and a second size deviation that is a difference in size between the second neighboring tooth and the second standard tooth.

In addition, the step of predicting a size of the mossing tooth is a step of predicting a size of the missing tooth by applying the first and second size deviations to the size of a third standard tooth of the standard tooth model corresponding to the missing tooth and located between the first and second standard teeth.

In addition, the step of predicting a size of the missing tooth is a step of predicting a size of the missing tooth by applying an intermediate value of the first and second size deviations to the size of the third standard tooth.

In addition, the present invention provides an apparatus for predicting a missing tooth in an oral image, including a model generation unit for generating a standard tooth model by statistically learning a plurality of training images; a whole-area matching unit for whole-area matching an oral image to the standard tooth model; and a prediction unit for predicting a missing tooth by comparing first and second neighboring teeth located on both sides of the missing tooth, respectively, with first and second standard teeth of the standard tooth model corresponding to the first and second neighboring teeth, respectively.

### [Advantageous Effects]

According to the present invention, by automatically predicting the central position, central axis direction and size information of a missing tooth, the crown and the implant can be automatically arranged to match the missing tooth area, thereby providing convenience to the operator during implant operation.

The effects obtainable in the present invention are not limited to the aforementioned effect, and other effects that are not mentioned may be clearly understood by those of ordinary skill in the art to which the present invention pertains from the following description.

### [Description of Drawings]

FIG. 1 is a schematic block diagram of the apparatus for predicting a missing tooth in an oral image according to an exemplary embodiment of the present invention.
FIG. 2 is a detailed block diagram of the control unit of FIG. 1.
FIG. 3 is a flowchart of the method for predicting a missing tooth in an oral image according to an exemplary embodiment of the present invention.
FIG. 4 is a detailed flowchart of the step for predicting a missing tooth in FIG. 3.
FIG. 5 is a diagram for describing the method of generating a training image according to an exemplary embodiment of the present invention.
FIGS. 6 to 8 are diagrams showing the central position, central axis and shape of individual teeth learned by the standard tooth model according to an exemplary embodiment of the present invention.
FIG. 9 is a diagram for describing the method of whole-area matching an oral image to a standard tooth model according to an exemplary embodiment of the present invention.
FIG. 10 is a diagram for describing the method of predicting the central position of a missing tooth according to an exemplary embodiment of the present invention.
FIG. 11 is a diagram for describing the method of predicting the central axis of a missing tooth according to an exemplary embodiment of the present invention.
FIG. 12 is a diagram for describing the method of predicting the size of a missing tooth according to an exemplary embodiment of the present invention.

### [Modes of the Invention]

The objects and means of the present invention and the effects therefrom will become more apparent from the following detailed description in conjunction with the accompanying drawings, and accordingly, the present invention will be easily practiced by those of ordinary skill in the art to which the present invention pertains. Further, in terms of describing the present invention, if it is determined that well-known technologies related to the present invention would obscure the gist of present invention due to unnecessary detail, the detailed description thereof will be omitted.

In the present specification, terms such as "or" and "at least one" may indicate one of the words listed together or a combination of two or more. For example, "or B" and "at least one of B" may include only one of A or B, or both A and B.

In the present specification, terms such as 'first' and 'second' may be used to describe various components, but the components should not be limited by the above terms. In addition, the above terms should not be construed as limiting the order of each component, and may be used for the purpose of distinguishing one component from another. For example, a 'first component' may be referred to as a 'second component', and similarly, a 'second component' may also be referred to as a 'first component.'

Hereinafter, preferred exemplary embodiments according to the present invention will be described in detail with reference to the accompanying drawings.

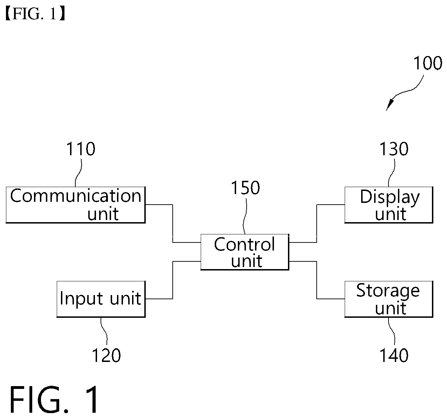

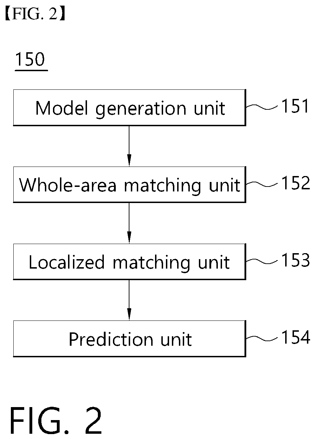

FIG. 1 is a schematic block diagram of the apparatus for predicting a missing tooth in an oral image according to an exemplary embodiment of the present invention, and FIG. 2 is a detailed block diagram of the control unit of FIG. 1.

The apparatus 100 for predicting a missing tooth in an oral image according to an exemplary embodiment of the present invention is an electronic apparatus that is executable by a computer program, and it is an apparatus for automatically predicting a missing tooth based on an oral image of the inside of the recipient's oral cavity.

Herein, the oral image may be an oral scan image, but is not limited thereto.

The oral scan image may provide information about the shape of the crown portion of a tooth exposed to the outside and the shape of the gum around the tooth. In this case, the oral scan image may be obtained by directly scanning the inside of the recipient's oral cavity through an oral scanner or the like, and or may be obtained by scanning an impression model that mimics the inside of the recipient's oral cavity with an intaglio or a plaster model created through embossing of the impression model, and the scanned image of the impression model may be inverted and used as an oral scan image.

Referring to FIG. 1, the apparatus 100 for predicting a missing tooth in an oral image according to an exemplary embodiment of the present invention may include a communication unit 110, an input unit 120, a display unit 130, a storage unit 140 and a control unit 150.

The communication unit 110 is a configuration for communicating with an external device such as an image acquisition device (not illustrated) and a server (not illustrated), and may receive image data. For example, the communication unit 110 may perform wireless communication such as 5^{th} generation communication (5G), long term evolution-advanced (LTE-A), long term evolution (LTE), Bluetooth, Bluetooth low energy (BLE), near field communication (NFC) and the like, and may perform wired communication such as cable communication and the like.

In this case, the image data may include other image data measured by other measurement methods in addition to the oral scan image data. For example, the other image data may include CT image data, MRI image data and the like.

The input unit 120 generates input data in response to a user's input, and may include at least one input means. For example, the input unit 120 may include a keyboard, a keypad, a dome switch, a touch panel, a touch key, a mouse, a menu button and the like.

The display unit 130 displays display data according to the operation of the oral image matching device 100. The display data may include image data. For example, the display unit 130 may include a liquid crystal display (LCD), a light emitting diode (LED) display, an organic light emitting diode (OLED) display, and a micro-electromechanical system (MEMS) display and an electronic paper display. In addition, the display unit 130 may be implemented as a touch screen in combination with the input unit 120.

The storage unit 140 stores various information and programs necessary for the operation of the apparatus 100 for predicting a missing tooth in an oral image. For example, the storage unit 140 may store image data received from an image acquisition device or the like and algorithms related to the method of predicting a missing tooth in an oral image according to an exemplary embodiment of the present invention, which will be described below. In addition, the storage unit 140 may store a standard tooth model obtained by learning the training image data by a deep learning method in order to predict a missing tooth.

The control unit 150 performs an operation of predicting a missing tooth with respect to the oral image data received from the image acquisition device or server, or pre-stored in the storage unit 140. To this end, the control unit 150 may receive the oral image data from the image acquisition device or the server and store it in the storage unit 140. In addition, the control unit 150 may control the operations of the communication unit 110, the input unit 120, the display unit 130 and the storage unit 140.

Referring to FIG. 2, the control unit 150 may be configured by including a model generation unit 151, a whole-area matching unit 152, a localized matching unit 153 and a prediction unit 154.

The model generation unit 151 generates a standard tooth model by statistically learning a plurality of training images by a deep learning method. Herein, the training image may be a matching image obtained by matching individual tooth models to an oral scan image, and the oral scan image may be an oral image without missing teeth.

The deep learning method is a supervised learning method, and may be one of the following methods of an artificial neural network, boosting, Bayesian statistics, decision tree, Gaussian process regression, nearest neighbor algorithm, support vector machine, random forests, symbolic machine learning, ensembles of classifiers, deep Learning and the like.

The whole-area matching unit 152 matches whole areas of the oral image of the recipient and the standard tooth model. Herein, the whole-area matching unit 152 matches all individual teeth included in the standard tooth model as a group.

The localized matching unit 153 locally matches the oral image and the standard tooth model, and the prediction unit 154 respectively compares first and second neighboring teeth located on both sides of a missing tooth with first and second standard teeth of the standard tooth model corresponding to the first and second neighboring teeth, and predicts the missing teeth (*e.g.,* the central position, central axis and size of a missing tooth). Herein, the localized matching unit 153 matches local areas of the oral image and the standard tooth model based on the first and second standard teeth.

That is, in the apparatus 100 for predicting a missing tooth in an oral image according to an exemplary embodiment of the present invention, the whole-area matching unit 152 primarily matches all individual teeth as a group, and then, the localized matching unit 153 secondarily matches a missing tooth and its neighboring teeth partially. Herein, in the primary matching, all individual teeth are matched while moving integrally, and in the secondary matching, only the missing teeth and neighboring teeth may be matched while moving.

In this way, the apparatus 100 for predicting a missing tooth in an oral image according to the present invention may automatically predict the central position, central axis and size of a missing tooth so as to automatically place a crown to match the missing tooth such that it is possible to provide convenience to the operator during implant operation.

Hereinafter, the method for predicting a missing tooth in an oral image according to an exemplary embodiment of the present invention controlled by the control unit 150 will be described.

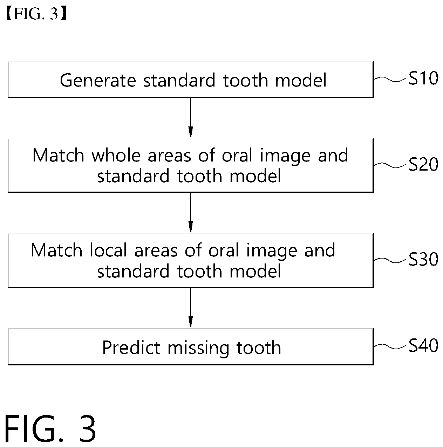

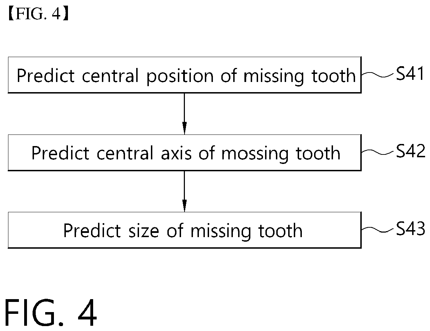

FIG. 3 is a flowchart of the method for predicting a missing tooth in an oral image according to an exemplary embodiment of the present invention, and FIG. 4 is a detailed flowchart of the step for predicting a missing tooth in FIG. 3.

Referring to FIG. 3, the method for predicting a missing tooth in an oral image according to an exemplary embodiment of the present invention may be configured by including the steps of generating a standard tooth model (S10), whole-area matching an oral image to the standard tooth model (S20), locally matching the oral image and the standard tooth model (S30) and predicting a missing tooth (S40).

In addition, referring to FIG. 4, the step of predicting a missing tooth (S40) may be configured by including the steps of predicting the central position of the missing tooth (S41), predicting the central axis of the missing tooth (S42) and predicting the size of the missing tooth (S43).

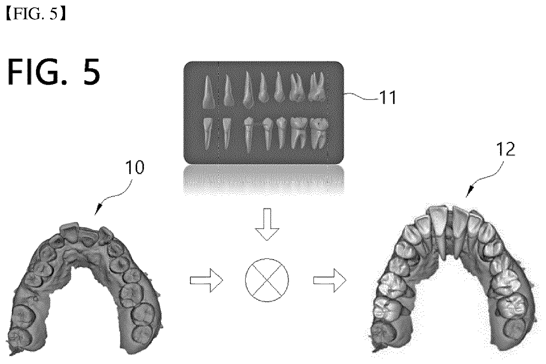

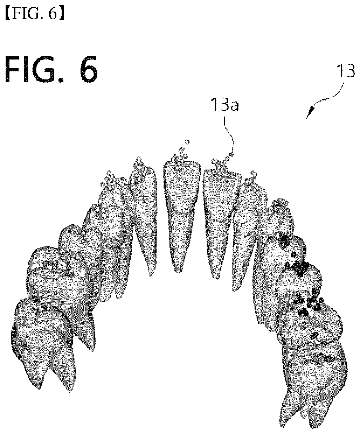

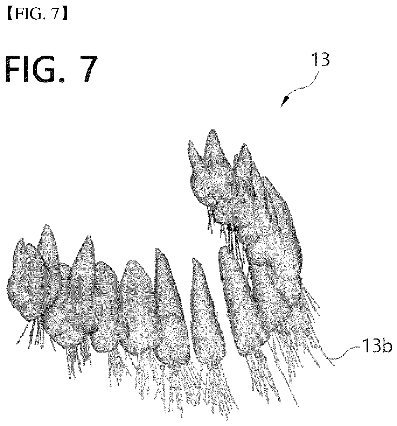

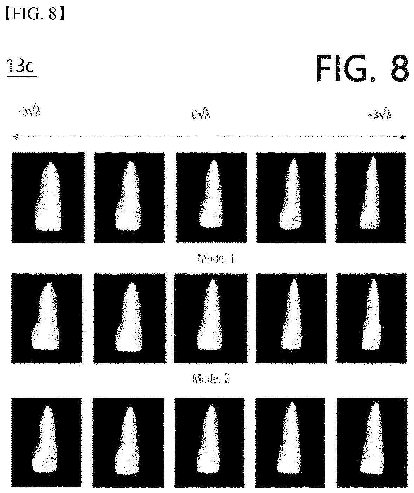

FIG. 5 is a diagram for describing the method of generating a training image according to an exemplary embodiment of the present invention, and FIGS. 6 to 8 are diagrams showing the central position, central axis and shape of individual teeth learned by the standard tooth model according to an exemplary embodiment of the present invention.

Referring to FIG. 5, a standard tooth model is generated by statistically learning a plurality of training images (S10). Herein, the standard tooth model 13 may be generated by learning the matching image 12 obtained by matching the individual tooth model 11 to the oral scan image 10 as the training image. In addition, the individual tooth model 11 may be composed of the different types of 14 individual teeth except wisdom teeth, and the standard tooth model 13 may be generated by aligning 14 individual teeth in the oral scan image 10.

Referring to FIGS. 6 to 8, the standard tooth model 13 is formed by learning the central position 13a, central axis 13b and shape 13c of individual teeth as parameters.

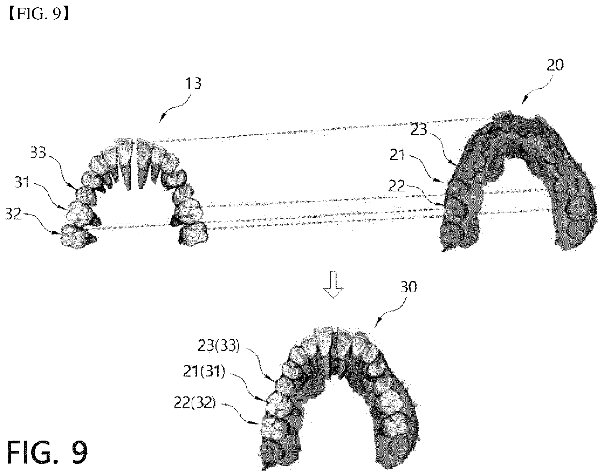

FIG. 9 is a diagram for describing the method of whole-area matching an oral image to a standard tooth model according to an exemplary embodiment of the present invention.

Referring to FIG. 9, a matching image 30 obtained by whole-area matching the oral image 20 of the recipient to the standard tooth model 13 is generated (S20). Herein, all individual teeth included in the standard tooth model 13 may be matched to the oral image 20 as a group. As such, when the oral image 20 and the standard tooth model 13 are matched, the matching image 30 may be generated.

Next, in the matching image 30, the first and second neighboring teeth 22, 23 located on both sides of a missing tooth 21 in the oral image 20 may be respectively compared to the first and second standard teeth 32, 33 of the standard tooth model 13 respectively corresponding to the first and second neighboring teeth 22, 23 so as to predict the missing tooth 21 (*e.g*., the central position, central axis and size of the missing tooth 21) (S40).

Meanwhile, after whole-area matching (S20), the method for predicting a missing tooth in an oral image according to an exemplary embodiment of the present invention may locally match the oral image 20 to the standard tooth model 13 based on the first and second standard teeth 32, 33 (S30).

As such, in the method for predicting a missing tooth in an oral image according to an exemplary embodiment of the present invention, after matching all individual teeth as a group, the missing tooth 21 and the neighboring teeth 22 and 23 adjacent thereto are secondarily matched partially. That is, in the primary matching, all individual teeth are matched while moving integrally, and in the secondary matching, only the missing tooth 21 and its neighboring teeth 22 and 23 are matched while moving. Herein, the meaning of moving is a relative concept and may be matched while the standard tooth model 13 moves or may be matched while the oral image 20 of the recipient moves.

The method for predicting a missing tooth in an oral image according to an exemplary embodiment of the present invention predicts the missing tooth 21 by using matching information of the oral image 20 and the standard tooth model 13, and in particular, the missing tooth 21 is predicted by using the matching information of its neighboring teeth 22, 23, by focusing on the point that the matching information of the missing tooth 21 has a certain tendency or similarity with the matching information of the neighboring teeth 22 and 23 adjacent to both sides thereof, respectively.

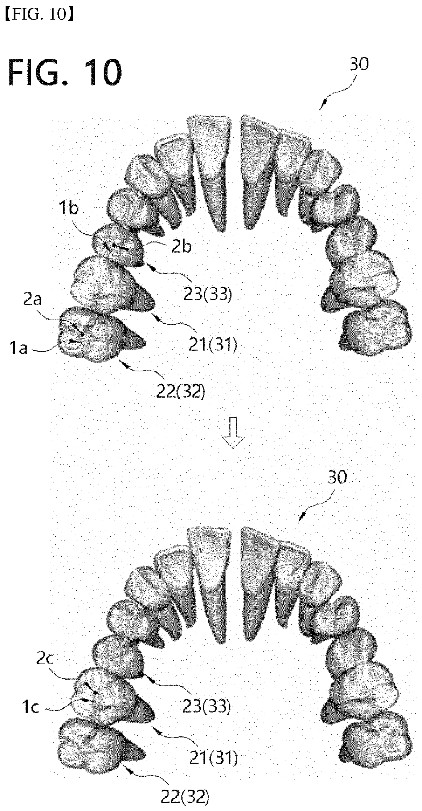

FIG. 10 is a diagram for describing the method of predicting the central position of a missing tooth according to an exemplary embodiment of the present invention.

Referring to FIG. 10, it can be confirmed that the central position 1a of the first neighboring tooth 22 and the central position 2a of the first standard tooth 32 are different, and the central position 1b of the second neighboring tooth 23 is different from the central position 2b of the second standard tooth 33.

Herein, based on a first position deviation that is a difference in the central positions between the first neighboring teeth 22 and the first standard teeth 32 and a second position deviation that is a difference in the central positions between the second neighboring teeth 23 and the second standard teeth 33, the central position 1c of the missing tooth 21 is predicted (S41).

Specifically, in the central position 2c of a third standard tooth 31 of the standard tooth model 13 corresponding to the missing tooth 21 and located between the first and second standard teeth 32 and 33, the central position 1c of the missing tooth 21 is predicted by applying the first and second position deviations.

For example, the central position 1c of the missing tooth 21 may be predicted by applying an intermediate value of the first and second positional deviations to the central position 2c of the third standard tooth 31. In this case, a weight may be assigned to the intermediate value according to the type of the missing tooth 21.

Herein, the central position of a tooth may be a two-dimensional or three-dimensional coordinate value.

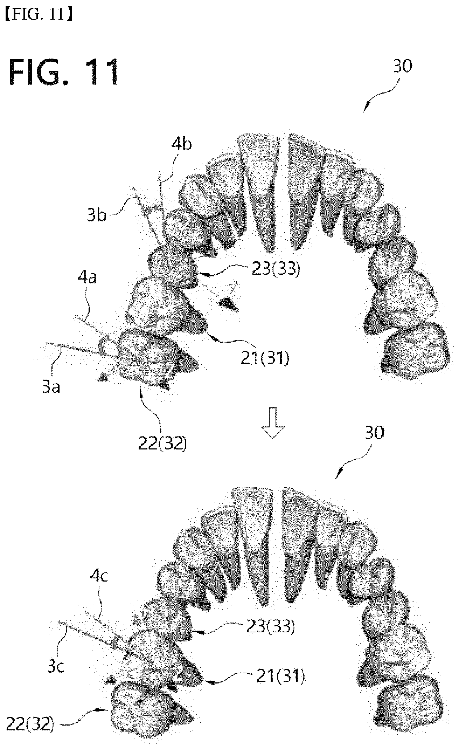

FIG. 11 is a diagram for describing the method of predicting the central axis of a missing tooth according to an exemplary embodiment of the present invention.

Referring to FIG. 11, it can be confirmed that the central axis 3a of the first neighboring tooth 22 and the central axis 4a of the first standard tooth 32 are different, and the central axis 3b of the second neighboring tooth 23 and the central axis 4b of the second standard tooth 33 are different.

Herein, based on a first angle deviation that is a central axis difference between the first neighboring teeth 22 and the first standard teeth 32 and a second angle deviation that is a central axis difference between the second neighboring teeth 23 and the second standard teeth 33, the central axis 3c of the missing tooth 21 is predicted (S42).

Specifically, in the central axis 4c of a third standard tooth 31 of the standard tooth model 13 corresponding to the missing tooth 21 and located between the first and second standard teeth 32, 33, the central axis 3c of the missing tooth 21 is predicted by applying the first and second angular deviations.

For example, the central axis 3c of the missing tooth 21 may be predicted by applying an intermediate value of the first and second angular deviations to the central axis 4c of the third standard tooth 31. In this case, a weight may be assigned to the intermediate value according to the type of the missing tooth 21.

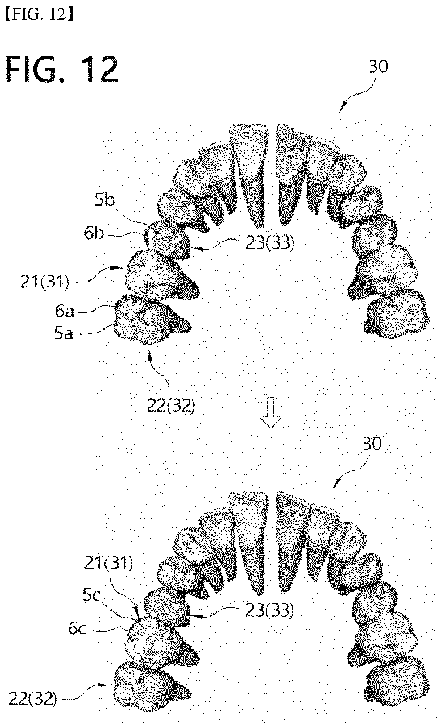

FIG. 12 is a diagram for describing the method of predicting the size of a missing tooth according to an exemplary embodiment of the present invention.

Referring to FIG. 12, it can be confirmed that the size 5a of the first neighboring tooth 22 and the size 6a of the first standard tooth 32 are different, and the size 5b of the second neighboring tooth 23 and the size 6b of the second standard tooth 33 are different.

Herein, based on the first size deviation that is a size difference between the first neighboring teeth 22 and the first standard teeth 32 and the second size deviation that is a size difference between the second neighboring teeth 23 and the second standard teeth 33, the size 5c of the missing tooth 21 is predicted (S43).

Specifically, by applying the first and second size deviations to the size 6c of a third standard tooth 31 of the standard tooth model 13 corresponding to the missing tooth 21 and located between the first and second standard teeth 32 and 33, the size 5c of the missing tooth 21 is predicted.

For example, the size 5c of the missing tooth 21 may be predicted by applying an intermediate value of the first and second size deviations to the size 6c of the third standard tooth 31. In this case, a weight may be assigned to the intermediate value according to the type of the missing tooth 21.

Herein, the size of a tooth may be the diameter of the upper portion of the tooth.

In this way, the apparatus 100 for predicting a missing tooth in an oral image according to the present invention may automatically detect the central position, central axis and size of a missing tooth and automatically place a crown to match the missing tooth such that it is possible to provide convenience to the operator during implant surgery.

In the detailed description of the present invention, although specific exemplary embodiments have been described, various modifications are possible without departing from the scope of the present invention. Therefore, the scope of the present invention is not limited to the described exemplary embodiments, and should be defined by the following claims and their equivalents.

### [Industrial Applicability]

The method and apparatus for predicting a missing tooth in an oral image according to the present invention may be used in various dental treatment fields such as implant surgery.

## Claims

1. A method for predicting a missing tooth in an oral image, comprising the steps of:
generating a standard tooth model by statistically learning a plurality of training images;
whole-area matching an oral image to the standard tooth model; and
predicting a missing tooth by comparing first and second neighboring teeth located on both sides of the missing tooth, respectively, with first and second standard teeth of the standard tooth model corresponding to the first and second neighboring teeth, respectively.

2. The method of claim 1, further comprising the step of:
locally matching the oral image and the standard tooth model based on the first and second standard teeth.

3. The method of claim 1, wherein the step of generating a standard tooth model is a step of generating the standard tooth model by learning a matching image obtained by matching an individual tooth model to an oral scan image.

4. The method of claim 1, wherein the step of whole-area matching is a step of matching all individual teeth included in the standard tooth model as a group.

5. The method of claim 1, wherein the step of predicting a missing tooth comprises the step of:
predicting a central position of the missing tooth based on a first position deviation that is a central position difference between the first neighboring tooth and the first standard tooth and a second position difference that is a central position difference between the second neighboring tooth and the second standard tooth.

6. The method of claim 5, wherein the step of predicting a central position of the missing tooth is a step of predicting a central position of the missing tooth by applying the first and second position deviations to the central position of a third standard tooth of the standard tooth model corresponding to the missing tooth and located between the first and second standard teeth.

7. The method of claim 5, wherein the step of predicting a central position of the missing tooth is a step of predicting a central position of the missing tooth by applying an intermediate value of the first and second positional deviations to the central position of the third standard tooth.

8. The method of claim 1, wherein the step of predicting a missing tooth comprises the step of:
predicting a central axis of the missing tooth based on a first angular deviation that is an angular difference between the central axes between the first neighboring tooth and the first standard tooth and a second angular deviation that is an angular difference between the central axes between the second neighboring tooth and the second standard tooth.

9. The method of claim 8, wherein the step of predicting a central axis of the missing tooth is a step of predicting a central axis of the missing tooth by applying the first and second angular deviations to the central axis of a third standard tooth of the standard tooth model corresponding to the missing tooth and located between the first and second standard teeth.

10. The method of claim 8, wherein the step of predicting a central axis of the missing tooth is a step of predicting a central axis of the missing tooth by applying an intermediate value of the first and second angular deviations to the central axis of the third standard tooth.

11. The method of claim 1, wherein the step of predicting a missing tooth comprises the step of:
predicting a size of the missing tooth based on a first size deviation that is a difference in size between the first neighboring tooth and the first standard tooth and a second size deviation that is a difference in size between the second neighboring tooth and the second standard tooth.

12. The method of claim 11, wherein the step of predicting a size of the mossing tooth is a step of predicting a size of the missing tooth by applying the first and second size deviations to the size of a third standard tooth of the standard tooth model corresponding to the missing tooth and located between the first and second standard teeth.

13. The method of claim 11, wherein the step of predicting a size of the missing tooth is a step of predicting a size of the missing tooth by applying an intermediate value of the first and second size deviations to the size of the third standard tooth.

14. An apparatus for predicting a missing tooth in an oral image, comprising:
a model generation unit for generating a standard tooth model by statistically learning a plurality of training images;
a whole-area matching unit for whole-area matching an oral image to the standard tooth model; and
a prediction unit for predicting a missing tooth by comparing first and second neighboring teeth located on both sides of the missing tooth, respectively, with first and second standard teeth of the standard tooth model corresponding to the first and second neighboring teeth, respectively.
